# EUROPÄISCHE PATENTSCHRIFT

(11) **EP 2 741 701 B1**
(45) Veröffentlichungstag und Bekanntmachung des Hinweises auf die Patenterteilung: **28.02.2018**
(21) Anmeldenummer: 12751466.9
(22) Anmeldetag: 09.08.2012
(51) Int. Cl.: A61B 17/86, A61B 17/80

(54) **SYSTEM AUS EINER KNOCHENPLATTE UND EINER KNOCHENSCHRAUBE**
SYSTEM COMPRISING A BONE PLATE AND A BONE SCREW
SYSTÈME COMPOSÉ D'UNE PLAQUE OSSEUSE ET D'UNE VIS OSSEUSE

(30) Priorität: 11.08.2011 EP 11177230
(43) Veröffentlichungstag der Anmeldung: 18.06.2014
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll
(86) Internationale Anmeldenummer: PCT/EP2012/065607
(87) Internationale Veröffentlichungsnummer: WO 2013/021033

(56) Entgegenhaltungen:
- DE-A1- 19 629 011
- DE-A1- 19 962 317
- DE-A1-102004 035 546
- US-A1- 2005 059 970
- US-A1- 2008 140 130

## Beschreibung

Die Erfindung betrifft ein System aus einer Knochenplatte und einer Knochenschraube. Die Knochenplatte ist mit einem Durchgangsloch versehen, das sich von einer Oberseite bis zu einer knochenseitigen Unterseite der Knochenplatte erstreckt. In dem Durchgangsloch ist eine Lippe ausgebildet, die von der Mantelfläche des Durchgangslochs vorspringt und die sich in Umfangsrichtung des Durchgangslochs erstreckt. Die Knochenschraube weist ein Kopfgewinde auf, das dazu ausgelegt ist, die Lippe in dem Durchgangsloch umzuformen, um eine Gewindeverbindung zu bilden.

Solche Knochenplatten sind dazu ausgelegt, mit einem Knochen verbunden zu werden. Sie können beispielsweise dazu dienen, einen Knochen nach einem Bruch zu stabilisieren. Dazu wird die Knochenplatte so positioniert, dass sie sich über die Bruchstelle hinweg erstreckt, und dann an den Knochenfragmenten befestigt. Die Bruchstelle wird dadurch ruhig gestellt und der Knochen kann ausheilen. Die Knochenplatte kann auch anderen Zwecken dienen und beispielsweise ein mit einem Knochen zu verbindendes Element einer Endoprothese sein.

Zur Befestigung der Knochenplatte an dem Knochen wird eine Knochenschraube in das Durchgangsloch eingesetzt, die sowohl am Schaft als auch am Kopf mit einem Gewinde versehen ist. Der Schaft der Schraube dringt so weit in das Knochenmaterial ein, dass der Kopf der Schraube in das Durchgangsloch der Knochenplatte gelangt. Der Kopf der Knochenschraube ist so bemessen, dass sein äußerer Durchmesser größer ist als der kleinste Durchmesser der Lippe. Beim weiteren Eindrehen der Knochenschraube wird die Lippe verformt, so dass sich eine Gewindeverbindung zwischen dem Kopf der Knochenschraube und der Lippe im Durchgangsloch der Knochenschraube bildet. Indem sowohl zwischen dem Schaft der Knochenschraube und dem Knochenmaterial als auch zwischen dem Kopf der Schraube und der Knochenplatte eine Gewindeverbindung besteht, wird eine sichere Verbindung zwischen der Knochenplatte und dem Knochen hergestellt. Da die Gewindeverbindung zwischen dem Kopf der Knochenschraube und der Knochenplatte erst beim Eindrehen der Knochenschraube durch einen Umformvorgang gebildet wird, ist es nicht erforderlich, die Knochenschraube in einem bestimmten vorgegebenen Winkel in die Knochenplatte einzudrehen. Vielmehr kann der Winkel innerhalb bestimmter Grenzen frei gewählt werden. Dies gibt den Chirurgen eine hohe Flexibilität während der Operation.
Bei bisherigen Knochenplatten muss das Kopfgewinde der Knochenschraube bereits kurz nach dem ersten Eingreifen eine größere Menge Material verdrängen, um das Gewinde zu bilden. Der Eingriff des Kopfgewindes in die Lippe wird dadurch erschwert. Ein System mit den Merkmalen des Oberbegriffs des unabhängigen Anspruchs ist aus der DE 199 62 317 A1 bekannt. Ein weiteres System ist aus der DE 10 2004 035546 A1 bekannt.

Der Erfindung liegt die Aufgabe zu Grunde, ein System aus einer Knochenplatte und einer Knochenschraube vorzustellen, bei dem die Knochenschraube leicht in die Lippe eingreifen kann. Ausgehend vom eingangs genannten Stand der Technik wird die Aufgabe gelöst mit den Merkmalen des unabhängigen Anspruchs. Vorteilhafte Ausführungsformen finden sich in den Unteransprüchen.

Erfindungsgemäß weist die Lippe eine zwischen der Mantelfläche des Durchgangslochs und einem zentralen Bereich der Lippe angeordnete Stufe auf, die sich längs der Lippe erstreckt.

Zunächst werden einige Begriffe erläutert. Eine Lippe bezeichnet einen Bereich, in dem die Materialstärke verglichen mit der Materialstärke der Knochenplatte vermindert ist. Die Lippe springt von der Mantelfläche des Durchgangslochs in Richtung des Zentrums des Durchgangslochs vor. Die Lippe hat eine Längserstreckung entlang dem Umfang des Durchgangslochs. Die Lippe erstreckt sich vorzugsweise ohne Unterbrechung über den gesamten Umfang des Durchgangslochs. Die Stufe kann sich über die gesamte Länge der Lippe erstrecken. Die Lippe hat eine in Richtung der Oberseite der Knochenplatte weisende Lippenoberfläche und eine in Richtung der Unterseite der Knochenplatte weisende Lippenunterfläche, die jeweils eine Ausdehnung in Umfangsrichtung und eine Ausdehnung in Radialrichtung haben. Die Unterseite der Knochenplatte liegt auf dem Knochen auf, wenn die Knochenplatte eingesetzt ist.

Wenn die Lippe mit einer Stufe versehen ist, bedeutet dies, dass, wenn man sich ausgehend von Zentrum in radialer Richtung nach außen bewegt, die Lippe im Bereich der Stufe eine größere Steigung hat als im Bereich des zentralen Abschnitts. Ausgehend vom Zentrum des Durchgangslochs folgt die Stufe in peripherer Richtung auf den zentralen Abschnitt. Die Steigung bezeichnet den Winkel, den die Lippenoberfläche bzw. Lippenunterfläche mit der sich quer zur Axialrichtung des Durchgangslochs erstreckenden Ebene einschließt.

Durch die Stufe wird es möglich, dass die Lippe im zentralen Bereich einen Abschnitt aufweist, in dem die Materialstärke gering ist. Die Knochenschraube, die in diesen Abschnitt der Lippe eingreift, muss zunächst nur wenig Material verdrängen. Trotzdem besteht unmittelbar nach dem ersten Eingriff eine sichere Führung, so dass die Knochenschraube sich beim weiteren Eindrehen entlang einer definierten Bahn bewegt. Erst wenn die Knochenschraube in den Bereich der Stufe vordringt, muss mehr Material verdrängt werden. Die Gewindeverbindung zwischen dem Kopf der Knochenschraube und der Lippe wird damit in zwei Schritten gebildet. In einem ersten Schritt greift das Kopfgewinde in einen Bereich geringer Materialstärke ein, so dass der erste Eingriff leicht und mit geringem Kraftaufwand stattfindet. Erst wenn das Kopfgewinde bis in den Bereich der Stufe vorgedrungen ist, wird so viel Material verdrängt, dass die Gewindeverbindung die hinreichende Stabilität erhält.

Die Lippe kann auf der Lippenoberfläche mit einer Stufe versehen sein. Alternativ oder zusätzlich dazu kann auf der Lippenunterfläche eine Stufe vorgesehen sein.

Von der Erfindung umfasst sind Ausführungsformen, bei denen das periphere Ende der Stufe in die Mantelfläche des Durchgangslochs übergeht. Die Steigung der Stufe ist dann vorzugsweise um mindestens 20°, vorzugsweise mindestens 30°, weiter vorzugsweise mindestens 45° kleiner als die Steigung der Mantelfläche des Durchgangslochs. Die Mantelfläche des Durchgangslochs kann eine Steigung von 90° haben. Damit oberhalb der Lippe ausreichend Platz für den Kopf der Knochenschraube bleibt, so dass die Knochenschraube ohne Kollision mit der Mantelfläche des Durchgangslochs in unterschiedlichen Winkeln eingeschraubt werden kann, kann die Steigung der Mantelfläche auch kleiner als 90° sein.

Alternativ kann die Lippe einen peripheren Bereich umfassen, der sich zwischen der Stufe und der Mantelfläche des Durchgangslochs erstreckt. Die Steigung im peripheren Bereich ist kleiner als die Steigung der Stufe. Wenn die Stufe einen Abstand zur Mantelfläche des Durchgangslochs hat, kann die Knochenschraube unter unterschiedlichen Winkeln in das Durchgangslochs eingesetzt werden, ohne dass es zu einer Kollision mit der Mantelfläche kommt.

Wenn das Kopfgewinde bis in der Bereich der Stufe vorgedrungen ist, besteht bereits ein sicherer Gewindeeingriff. Es besteht also keine Gefahr mehr, dass das Kopfgewinde an der Lippe abrutscht, auch wenn das Kopfgewinde nun in die steilere Stufe eingreifen muss. Der Chirurg spürt die Stufe als erheblich vergrößerten Widerstand beim Eindrehen der Schraube. Wenn die Steigung der Stufe hinreichend groß ist, reicht ab diesem Punkt ein begrenzter Drehwinkel aus, um die endgültige Gewindeverbindung zu bilden. Dies erhöht die Bedienungsfreundlichkeit, weil für den Chirurgen eine Anweisung, die Knochenschraube nach Erhöhung des Widerstands noch um beispielsweise 90° oder 180° weiter zu drehen, einfacher zu befolgen ist als ein bestimmtes vorgegebenes Drehmoment einzuhalten. In einer bevorzugten Ausführungsform ist die Steigung der Stufe deswegen größer als 45°, vorzugsweise größer als 60°, weiter vorzugsweise größer als 75°.

Der zentrale Bereich der Lippe dient in erster Linie dazu, einen leichten Eingriff des Kopfgewindes zu ermöglichen, während das Kopfgewinde seinen eigentlichen Halt in den weiter außen liegenden Teilen der Lippe findet. Für den ersten Eingriff ist es ausreichend, wenn der zentrale Bereich nur einen kleinen Teil der Lippe ausmacht. Vorzugsweise ist die Strecke vom zentralen Ende der Lippe bis zum zentralen Ende der Stufe nicht größer als 50 %, weiter vorzugsweise nicht größer als 30 %, weiter vorzugsweise nicht größer als 20 % als die Strecke vom zentralen Ende der Lippe bis zur Mantelfläche des Durchgangslochs.

Die Steigung der Stufe sollte deutlich größer sein als die Steigung im zentralen Bereich. Vorzugsweise ist die Steigung der Stufe um mindestens 20°, vorzugsweise um mindestens 30°, weiter vorzugsweise um mindestens 45° größer als die Steigung des zentralen Bereichs. Die Steigung im zentralen Bereich kann beispielsweise zwischen 0° und 40°, vorzugsweise zwischen 20° und 30° liegen. Die Stufe kann beispielsweise eine Steigung zwischen 60° und 90° haben. Wenn die Lippe einen peripheren Bereich umfasst, kann die Steigung dort ebenfalls zwischen 0° und 40°, vorzugsweise zwischen 20° und 30° liegen.

Um hinreichend leicht verformbar zu sein, ist die Lippe vorzugsweise auch an ihrer dicksten Stelle deutlich dünner als die Knochenplatte. Vorzugsweise erstreckt sich die dickste Stelle der Lippe sich über nicht mehr als 80 %, vorzugsweise nicht mehr als 60 %, weiter vorzugsweise nicht mehr als 50 % der Länge des Durchgangslochs.

Von Vorteil ist es, wenn die in die Knochenplatte eingeschraubte Knochenschraube möglichst wenig über die Knochenplatte hinausragt, weil ansonsten die Gefahr besteht, dass das umliegende Gewebe irritiert wird. Wenn die Lippe in der an die Unterseite der Knochenplatte angrenzenden (unteren) Hälfte des Durchgangslochs angeordnet ist, ist oberhalb der Lippe Raum, um den Kopf der Knochenschraube in der Knochenplatte zu versenken. Die an die Oberseite der Knochenplatte angrenzende (obere) Hälfte des Durchgangslochs wird dann von der Mantelfläche des Durchgangslochs gebildet, ist also frei von der Lippe.

Die Knochenplatte mit der Lippe in dem Durchgangsloch kann einstückig ausgebildet sein. Alternativ ist es möglich, dass ein das Durchgangsloch umgebender Bereich in Form eines Inlays in die Knochenplatte eingesetzt ist. Das Inlay mit der Lippe kann zunächst als separates Teil gebildet werden und dann mit der Knochenplatte verbunden werden. Um das Formen der Gewindeverbindung zu erleichtern, kann das Inlay aus einem weicheren Material bestehen als die Knochenplatte.

Um dem Chirurgen wahlweise die Verwendung konventioneller Knochenschrauben zu erlauben, deren Kopf nicht mit einem Gewinde versehen ist, kann die Mantelfläche des Durchgangslochs oberhalb der Lippe nach außen aufgeweitet sein. Dieser Bereich der Mantelfläche kann dann eine Gegenfläche für einen konventionellen Schraubenkopf bilden. Im Querschnitt kann die Aufweitung beispielsweise die Form eines Kreissegments haben.

Die Erfindung betrifft außerdem ein System aus einer solchen Knochenplatte und einer Knochenschraube. Die Knochenschraube hat ein Kopfgewinde, das dazu ausgelegt ist, die Lippe in dem Durchgangsloch umzuformen, um eine Gewindeverbindung zu bilden. Um eine sichere Befestigung an dem Knochen zu ermöglichen, hat die Knochenplatte in aller Regel eine Mehrzahl von Durchgangslöchern. Entsprechend kann auch das System eine Mehrzahl von Knochenschrauben umfassend.

Die beim Bilden der Gewindeverbindung stattfindende Materialumformung soll möglichst nicht mit Spanabrieb verbunden sein. Versuche haben gezeigt, dass der Spanabrieb gering gehalten wird, wenn der Schraubenkopf eine kegelförmige Mantelfläche aufweist und der Winkel, den die Mantelfläche mit der Schraubenachse einschließt, zwischen 19° und 28° liegt.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine Ansicht von oben auf eine erfindungsgemäße Knochenplatte;
- Fig. 2:: einen Schnitt durch Fig. 1 entlang der Linie I-I;
- Fig. 3:: eine vergrößerte Ansicht des Details A aus Fig. 1;
- Fig. 4 bis 7:: unterschiedliche Ausführungsformen erfindungsgemäßer Lippen;
- Fig. 8 und 9:: die Ansicht aus Fig. 2 bei weiteren Ausführungsformen der Erfindung; und
- Fig. 10:: eine für die erfindungsgemäße Knochenplatte bestimmte Knochenschraube.

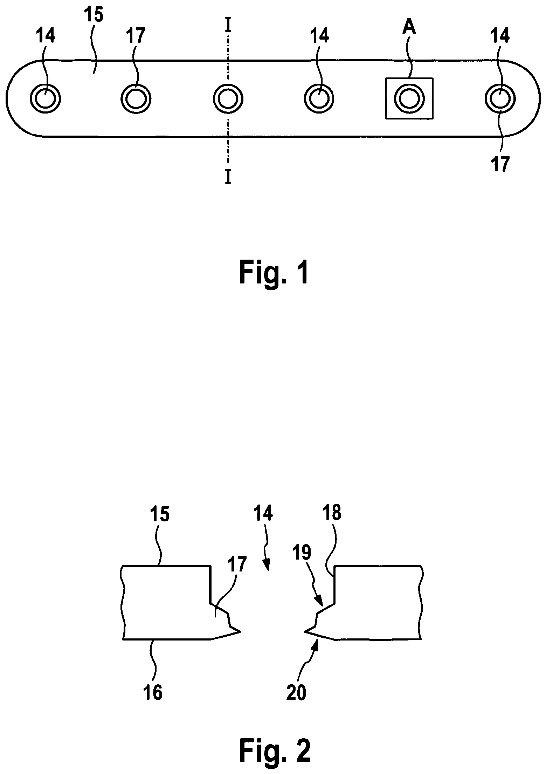

Eine erfindungsgemäße Knochenplatte hat gemäß den Figuren 1 und 2 sechs Durchgangslöcher 14, die sich von einer Oberseite 15 der Knochenplatte bis zur einer Unterseite 16 der Knochenplatte erstrecken. In jedem Durchgangsloch 14 ist eine Lippe 17 ausgebildet, die von der Mantelfläche 18 in Richtung des Zentrums des Durchgangslochs 14 vorspringt. Die Lippe 17 ist eine durchgehende Lippe, die sich ohne Unterbrechung über den gesamten Umfang des Durchgangslochs erstreckt. Die Lippe 17 hat eine zur Oberseite 15 der Knochenplatte weisende Lippenoberfläche 19 und eine zur Unterseite 16 der Knochenplatte weisende Lippenunterfläche 20. Die Lippe 17 schließt mit der Unterseite 16 der Knochenplatte ab, so dass oberhalb der Lippe 17 möglichst viel Raum für die Aufnahme des Schraubenkopfs bleibt.

Die Knochenplatte ist dazu bestimmt mit der Unterseite 16 auf einen Knochen aufgelegt zu werden, so dass die Knochenplatte sich über eine Bruchstelle des Knochens hinweg erstreckt. Nachdem der Knochen in die richtige Stellung reponiert ist, wird die Knochenplatte mit den Knochenfragmenten verbunden. Der Knochen ist dann in dieser Stellung fixiert und kann ausheilen.

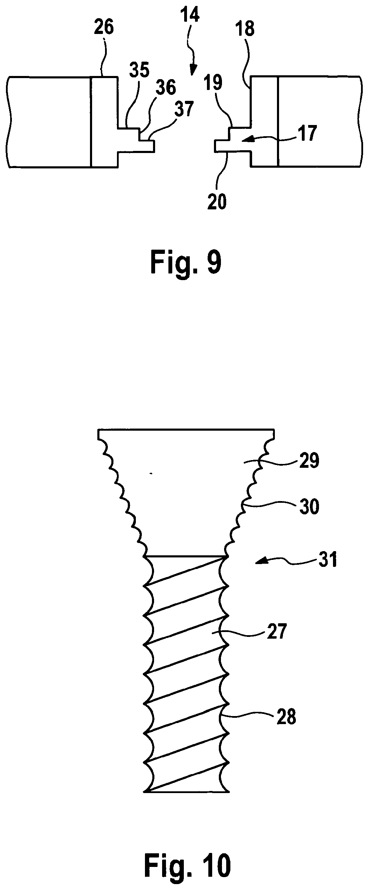

Zum Verbinden der Knochenplatte mit dem Knochen werden Knochenschrauben 31 verwendet, wie sie in Fig. 10 dargestellt sind. Die Knochenschrauben 31 haben einen Schaft 27, der mit einem Knochengewinde 28 versehen ist, und einen Schraubenkopf 29, dessen Außenfläche ein Kopfgewinde 30 mit kleinerer Steigung aufweist. Die Mantelfläche des Schraubenkopfs 29 schließt mit der Schraubenachse einen Winkel von 25° ein. Die Knochenschraube wird in den Knochen eingedreht, bis der Schraubenkopf 29 in das Durchgangsloch 14 eindringt. Dabei kann der Winkel, den die Knochenschraube mit der Achse des Durchgangslochs einschließt, zwischen etwa 0° und 15° frei gewählt werden. Wenn die Knochenschraube nun weiter eingedreht wird, kommt das Kopfgewinde 30 der Knochenschraube 31 in Eingriff mit der Lippe 17. Die Lippe 17 wird verformt und es wird eine Gewindeverbindung zwischen dem Kopf 29 der Knochenschraube 31 und der Lippe 17 gebildet. Um das Bilden der Gewindeverbindung zu erleichtern, kann die Knochenschraube 31 aus einem härteren Material bestehen als die Knochenplatte. Beispielsweise kann die Knochenplatte aus reinem Titan gefertigt sein, während die Knochenschraube 31 aus der Titanlegierung TiAl6V4 besteht.

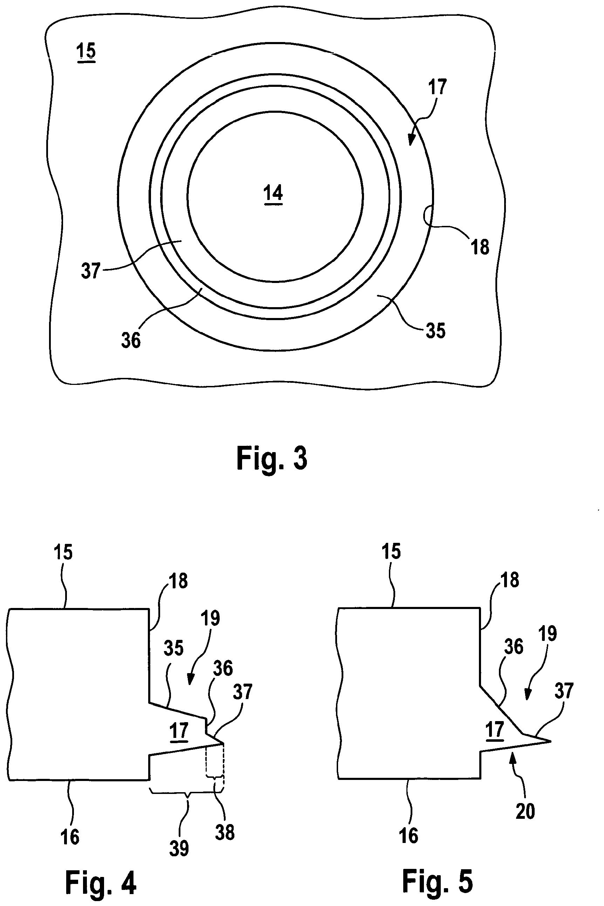

Gemäß Fig. 3 umfasst die Lippe 17 einen peripheren Bereich 35, eine Stufe 36 und einen zentralen Bereich 37. Die Lippe und die Bereiche 35, 36, 37 erstrecken sich über den gesamten Umfang des Durchgangslochs 14. Im peripheren Bereich 35 und im zentralen Bereich 37 ist die Steigung der Lippenoberfläche klein und beträgt beispielsweise etwa 15°. Im Bereich der Stufe 36, die den zentralen Bereich 37 und den peripheren Bereich 35 verbindet, ist die Steigung wesentlich größer und beträgt beispielsweise 75°.

Wenn eine Knochenschraube 31 in das Durchgangsloch 14 eingedreht wird, kommt das Kopfgewinde 30 zunächst mit dem zentralen Bereich 37 der Lippe 17 in Eingriff. Im zentralen Bereich 37 ist die Lippe dünn, so dass nur wenig Material verdrängt wird, wenn das Kopfgewinde 30 eindringt. Der Chirurg, der die Schraube eindreht, merkt kaum einen erhöhten Widerstand. Beim weiteren Eindrehen dringt das Kopfgewinde 30 weiter in den zentralen Bereich 37 ein und nähert sich an die Stufe 36 an. Wenn das Kopfgewinde 30 bei der Stufe 36 ankommt, ist das Gewinde bereits soweit ausgebildet, dass es eine sichere Führung für die Knochenschraube bietet. Wenn das Kopfgewinde 30 an der Stufe 36 anliegt, erhöht sich der Widerstand, der beim weiteren Eindrehen der Schraube überwunden werden muss, da nun eine größere Menge an Material umgeformt werden muss. Die Knochenschraube 31 wird ab dem Punkt des erhöhten Widerstands noch um 90° weiter eingedreht, so dass sich eine über den gesamten Umfang der Knochenschraube 31 erstreckende Gewindeverbindung bildet. Damit ist der Endzustand erreicht und das Kopfgewinde 30 ist sicher in der Lippe 17 verriegelt.

In Fig. 4 ist eine Ausführungsform gezeigt, bei der die Stufe 36 eine Steigung von 90° hat. Die Stufe 36 ist also parallel zur Mantelfläche 18 des Durchgangslochs 14. Der zentrale Bereich 37 der Lippe 17 macht lediglich den kleineren Teil der Lippe 17 aus. Der Abstand 38 zwischen dem zentralen Ende der Lippe und der Stufe 36 beträgt nur etwa ein Viertel des Abstands 39 zwischen dem zentralen Ende der Lippe 17 und der Mantelfläche 18 des Durchgangslochs 14.

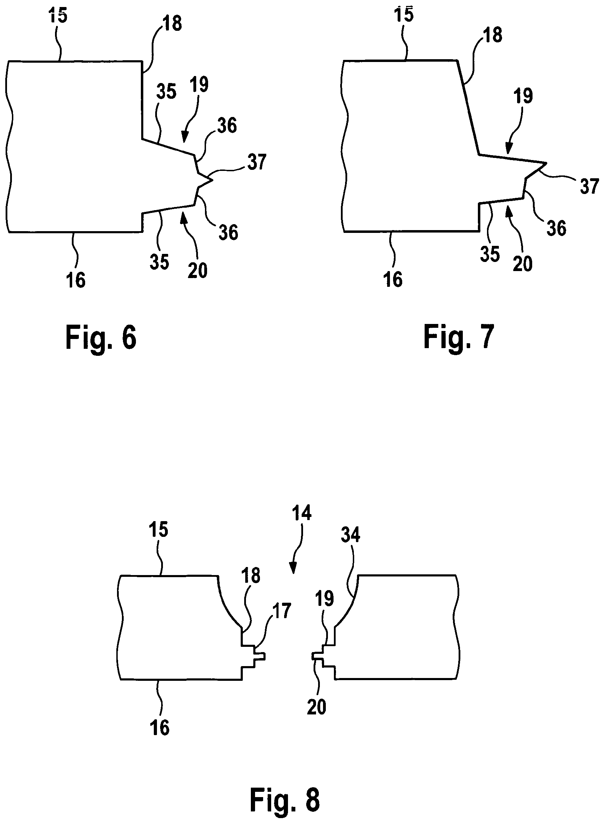

Bei der Ausführungsform der Fig. 5 besteht die Lippe 17 nur aus einem zentralen Bereich 37 und einer Stufe 36. Die Stufe 36 erstreckt sich vom peripheren Ende des zentralen Bereich 37 bis zur Mantelfläche 18 des Durchgangslochs 14. In Fig. 6 ist sowohl die Lippenoberfläche 19 als auch die Lippenunterfläche 20 mit einer Stufe 36 versehen. An die Stufe 36 schließt sich sowohl auf der Lippenoberfläche 19 als auch auf der Lippenunterfläche 20 ein peripherer Bereich 35 an, in dem die Steigung deutlich kleiner ist als bei der Stufe 36. Bei der Ausführungsform der Fig. 7 hat nur die Lippenunterfläche 20 eine Stufe 36. Die Mantelfläche 18 des Durchgangslochs 14 ist etwas nach außen geneigt, so dass mehr Raum für die Aufnahme des Schraubenkopfs bleibt.

Die Fig. 9 zeigt eine Ausführungsform, bei der ein Inlay 26 in die Knochenplatte eingesetzt ist. Das Inlay 26 besteht aus einem weicheren Material als die Knochenplatte und bildet die Lippe 17 sowie die Umgebung des Durchgangslochs 14. die Lippe umfasst einen zentralen Bereich 37 und einen peripheren Bereich 35, in denen die Steigung 0° beträgt. Die Stufe 36 hat eine Steigung von 90°.

In Fig. 8 hat die Mantelfläche 18 oberhalb der Lippe 17 eine Aufweitung 34, die im Querschnitt die Form eines Kreissegments hat. Bei dieser Ausführungsform besteht wahlweise die Möglichkeit der Verwendung konventioneller Knochenschrauben, deren Kopf frei von einem Gewinde ist. Die Aufweitung 34 bildet dann eine Gegenfläche für eine Knochenschraube mit einem halbkugelförmigen Kopf, so dass die Knochenschraube unter unterschiedlichen Winkeln in das Durchgangsloch 14 eingesetzt werden kann.

## Patentansprüche

1. System aus einer Knochenplatte und einer Knochenschraube (31), wobei die Knochenplatte mit einem Durchgangsloch (14) versehen ist, das sich von einer Oberseite (15) bis zu einer knochenseitigen Unterseite (16) der Knochenplatte erstreckt, wobei in dem Durchgangsloch (14) eine Lippe (17) ausgebildet ist, die von der Mantelfläche (18) des Durchgangslochs (14) vorspringt und sich in Umfangsrichtung des Durchgangslochs (14) erstreckt, und wobei die Knochenschraube (31) ein Kopfgewinde (30) aufweist, das dazu ausgelegt ist, die Lippe (17) in dem Durchgangsloch (14) umzuformen, um eine Gewindeverbindung zu bilden, **dadurch gekennzeichnet, dass** die Lippe (17) eine zwischen der Mantelfläche (18) des Durchgangslochs (14) und einem zentralen Bereich (37) der Lippe (17) angeordnete Stufe (36) aufweist, die sich längs der Lippe (17) erstreckt.

2. System nach Anspruch 1, **dadurch gekennzeichnet, dass** die Lippenoberfläche (19) mit einer Stufe (36) versehen ist.

3. System nach Anspruch 1 oder 2, **dadurch gekennzeichnet, dass** die Lippenunterfläche (20) mit einer Stufe (36) versehen ist.

4. System nach einem der Ansprüche 1 bis 3, **dadurch gekennzeichnet, dass** die Lippe (17) einen peripheren Bereich (35) umfasst, der sich zwischen der Stufe (36) und der Mantelfläche (18) des Durchgangslochs (14) erstreckt.

5. System nach einem der Ansprüche 1 bis 4, **dadurch gekennzeichnet, dass** die Steigung der Stufe (36) größer ist als 45°, vorzugsweise größer ist als 60°, weiter vorzugsweise größer ist als 75°.

6. System nach einem der Ansprüche 1 bis 5, **dadurch gekennzeichnet, dass** der Abstand (38) zwischen dem zentralen Ende der Lippe (17) und dem zentralen Ende der Stufe (36) nicht größer als 50 %, vorzugsweise nicht größer als 30 %, weiter vorzugsweise nicht größer als 20 % ist als der Abstand zwischen dem zentralen Ende der Lippe (17) und der Mantelfläche (18) des Durchgangslochs (14).

7. System nach einem der Ansprüche 1 bis 6, **dadurch gekennzeichnet, dass** die Steigung der Stufe (36) um mindestens 20°, vorzugsweise um mindestens 30°, weiter vorzugsweise um mindestens 45° größer ist als die Steigung des zentralen Bereichs (37).

8. System nach einem der Ansprüche 1 bis 7, **dadurch gekennzeichnet, dass** die Lippe (17) sich in Axialrichtung über nicht mehr als 80 %, vorzugsweise über nicht mehr als 60 %, weiter vorzugsweise über nicht mehr als 50 % der Länge des Durchgangslochs (14) erstreckt.

9. System nach einem der Ansprüche 1 bis 8, **dadurch gekennzeichnet, dass** die Lippe in der an die Unterseite (16) der Knochenplatte angrenzenden Hälfte des Durchgangslochs (14) angeordnet ist.

10. System nach einem der Ansprüche 1 bis 9, **dadurch gekennzeichnet, dass** ein das Durchgangsloch (14) umgebender Bereich in Form eines Inlays (26) in die Knochenplatte eingesetzt ist.

11. System nach einem der Ansprüche 1 bis 10, **dadurch gekennzeichnet, dass** die Knochenschraube (31) einen Kopf (29) mit einer kegelförmigen Mantelfläche aufweist und dass die Mantelfläche mit der Schraubenachse einen Winkel zwischen 19° und 28° einschließt.

## Claims

1. System comprising a bone plate and a bone screw (31), wherein the bone plate is provided with a through-hole (14) that extends from an upper face (15) of the bone plate to a lower face (16) thereof situated toward the bone, wherein a lip (17) is formed in the through-hole (14), which lip (17) protrudes from the curved surface (18) of the through-hole (14) and extends in the circumferential direction of the through-hole (14), and wherein the bone screw (31) has a head thread (30), which is designed to reshape the lip (17) in the through-hole (14) in order to form a threaded connection, **characterized in that** the lip (17) has a step (36) arranged between the curved surface (18) of the through-hole (14) and a central area (37) of the lip (17), which step (36) extends along the lip (17).

2. System according to Claim 1, **characterized in that** the lip top surface (19) is provided with a step (36).

3. System according to Claim 1 or 2, **characterized in that** the lip underside (20) is provided with a step (36).

4. System according to one of Claims 1 to 3, **characterized in that** the lip (17) comprises a peripheral area (35), which extends between the step (36) and the curved surface (18) of the through-hole (14) .

5. System according to one of Claims 1 to 4, **characterized in that** the gradient of the step (36) is greater than 45°, preferably greater than 60°, more preferably greater than 75°.

6. System according to one of Claims 1 to 5, **characterized in that** the distance (38) between the central end of the lip (17) and the central end of the step (36) is not greater than 50%, preferably not greater than 30%, more preferably not greater than 20% of the distance between the central end of the lip (17) and the curved surface (18) of the through-hole (14).

7. System according to one of Claims 1 to 6, **characterized in that** the gradient of the step (36) is at least 20°, preferably at least 30°, more preferably at least 45° greater than the gradient of the central area (37).

8. System according to one of Claims 1 to 7, **characterized in that** the lip (17) extends in the axial direction by not more than 80%, preferably by not more than 60%, more preferably by not more than 50% of the length of the through-hole (14).

9. System according to one of Claims 1 to 8, **characterized in that** the lip is arranged in the half of the through-hole (14) adjacent to the lower face (16) of the bone plate.

10. System according to one of Claims 1 to 9, **characterized in that** an area surrounding the through-hole (14) is inserted in the form of an inlay (26) into the bone plate.

11. System according to one of Claims 1 to 10, **characterized in that** the bone screw (31) has a head (29) with a conical curved surface, and **in that** the curved surface encloses an angle of between 19° and 28° with the axis of the screw.

## Revendications

1. Système composé d'une plaque osseuse et d'une vis à os (31), dans lequel la plaque osseuse est dotée d'un trou de passage (14), qui s'étend d'un côté supérieur (15) jusqu'à un côté inférieur côté os (16) de la plaque osseuse, dans lequel une lèvre (17) est formée dans le trou de passage (14), laquelle est saillante de la surface latérale (18) du trou de passage (14) et s'étend en direction périphérique du trou de passage (14), et dans lequel la vis à os (31) présente un filet de tête (30), qui est conçu pour déformer la lèvre (17) dans le trou de passage (14), afin de former un assemblage vissé, **caractérisé en ce que** la lèvre (17) présente un gradin (36) disposé entre la surface latérale (18) du trou de passage (14) et une région centrale (37) de la lèvre (17), et qui s'étend le long de la lèvre (17).

2. Système selon la revendication 1, **caractérisé en ce que** la face supérieure (19) de la lèvre est dotée d'un gradin (36).

3. Système selon la revendication 1 ou 2, **caractérisé en ce que** la face inférieure (20) de la lèvre est dotée d'un gradin (36).

4. Système selon l'une quelconque des revendications 1 à 3, **caractérisé en ce que** la lèvre (17) comprend une région périphérique (35), qui s'étend entre le gradin (36) et la surface latérale (18) du trou de passage (14).

5. Système selon l'une quelconque des revendications 1 à 4, **caractérisé en ce que** la pente du gradin (36) est supérieure à 45°, de préférence est supérieure à 60°, et de préférence encore est supérieure à 75°.

6. Système selon l'une quelconque des revendications 1 à 5, **caractérisé en ce que** la distance (38) entre l'extrémité centrale de la lèvre (17) et l'extrémité centrale du gradin (36) n'est pas supérieure à 50 %, de préférence n'est pas supérieure à 30 %, et de préférence encore n'est pas supérieure à 20 % de la distance entre l'extrémité centrale de la lèvre (17) et la surface latérale (18) du trou de passage (14).

7. Système selon l'une quelconque des revendications 1 à 6, **caractérisé en ce que** la pente du gradin (36) est supérieure d'au moins 20°, de préférence d'au moins 30°, et de préférence encore d'au moins 45° à la pente de la région centrale (37).

8. Système selon l'une quelconque des revendications 1 à 7, **caractérisé en ce que** la lèvre (17) ne s'étend en direction axiale pas sur plus de 80 %, de préférence pas sur plus de 60 %, et de préférence encore pas sur plus de 50 % de la longueur du trou de passage (14).

9. Système selon l'une quelconque des revendications 1 à 8, **caractérisé en ce que** la lèvre est disposée dans la moitié du trou de passage (14) adjacente au côté inférieur (16) de la plaque osseuse.

10. Système selon l'une quelconque des revendications 1 à 9, **caractérisé en ce qu'**une région entourant le trou de passage (14) est insérée sous la forme d'un inlay (26) dans la plaque osseuse.

11. Système selon l'une quelconque des revendications 1 à 10, **caractérisé en ce que** la vis à os (31) présente une tête (29) avec une surface latérale de forme conique et **en ce que** la surface latérale forme avec l'axe de la vis un angle situé entre 19° et 28°.
